# EUROPEAN PATENT SPECIFICATION

(11) **EP 1 964 853 B1**
(45) Date of publication and mention of the grant of the patent: **26.08.2009**
(21) Application number: 08250666.8
(22) Date of filing: 27.02.2008
(51) Int. Cl.: C07K 17/02, C12M 1/40

(54) **Methods of synthesizing and preserving a nucleotide-labeled microtubule**
Verfahren zum Synthetisieren und Aufbewahren eines nukleotidmarkierten Mikrotubulus
Procédés pour synthétiser et préserver un microtubule à étiquette nucléotide

(30) Priority: 27.02.2007 JP 2007047933
(43) Date of publication of application: 03.09.2008
(73) Proprietor: NTT DoCoMo, Inc., Chiyoda-ku Tokyo 100-6150 (JP); The University of Tokyo, Bunkyo-ku, Tokyo 113-8654 (JP)
(72) Inventor: Hiyama, Satoshi, c/o IPD, NTT DoCoMo, Inc., Tokyo 100-6150 (JP); Moritani, Yuki, c/o IPD, NTT DoCoMo, Inc., Tokyo 100-6150 (JP); Suda, Tatsuya, c/o IPD, NTT DoCoMo, Inc., Tokyo 100-6150 (JP); Sutoh, Kazuo, The University of Tokyo, Tokyo 113-8654 (JP)
(74) Representative: Lord, Hilton David

(56) References cited:
- EP-A- 1 798 282
- WO-A-20/04096831
- WO-A-20/06034128
- US-B1- 6 316 003
- HIYAMA S ET AL: "A Design of an Autonomous Molecule Loading/Transporting/Unloading System Using DNA Hybridization and Biomolecular Linear Motors" EUROPEAN NANO SYSTEMS. ENS PROCEEDINGS, X, XX, 14 December 2005 (2005-12-14), pages 75-80, XP009087172

## Description

### FIELD OF THE INVENTION

The present invention relates to a method of synthesizing a microtubule in general, and particularly, to a method of synthesizing a microtubule labeled with nucleotides (DNAs or RNAs) without using a biotin-avidin binding and also to methods of preserving and resynthesizing the produced microtubule.

### BACKGROUND OF THE INVENTION

In Eukaryotic cells, microtubules and actin filaments which are cytoskeleton are developed and used as the rails on which cargo molecules such as intracellular organelles or transport vesicles are transported by motor proteins such as kinesins or dyneins. A mechanism of a molecular transport which is represented by an interaction between kinesins and the microtubules can be reconstituted in vitro. A gliding assay system is also widely known, where the microtubules are caused to glide on kinesins immobilized on a substrate.

As an attempt to engineer this superior biological function to create an artificial molecular transport system, the surface of the gliding microtubules is biochemically modified for functional utilization (see, for example, Non-Patent Document 1 listed below). In this attempt, by biotinylating a portion of the surface of the gliding microtubules and allowing the biotinylated surface to be bound to the cargo molecules such as microbeads, which are covered with streptavidin, via a biotin-avidin binding, the cargo molecules have been successfully loaded and transported on the microtubules. However, this biotin-avidin binding is widely known to be one of the bindings having the highest affinity in interaction of biological systems, so there is a problem that once the cargo molecules are loaded on the microtubules, it is essentially impossible to unload them from the microtubules.

In contrast, a system is disclosed in which a microtubule labeled with a single stranded DNA is produced by biotinylating a portion of the surface of the gliding microtubule and causing the biotinylated surface to be bound to the single stranded DNA, of which the end is modified with streptavidin, via the biotin-avidin binding. Loading is performed by hybridization of a DNA having base sequences complementary to the single stranded DNA bound to the microtubule, and unloading is performed by a DNA cleavage caused by an addition of restriction enzymes (see, for example, Patent Documents 1 and 2 listed below).

However, since the single stranded DNA can be bound only to the biotinylated site and avidins and streptavidins are molecules whose molecular weights are large (60-67 kDa), it leads to a problem that a modification ratio (labeling stoichiometry) of the single stranded DNAs to the microtubules cannot be set arbitrarily. Therefore, if the labeling stoichiometry is higher than a certain amount, there is a concern that the gliding movement of the microtubules will be interfered with.

Prior to the filing of this patent application, the inventors proposed DNA-labeled microtubules considering the use of the biotin-avidin binding as well as a chemical crosslinking agent, and they have proposed a system, where a mechanism of selective and autonomous loading and unloading of cargo molecules is accomplished only by hybridization (Japanese Patent Application No. 2005-307880 yet to be published). However, this proposal does not refer to a specific method of synthesizing the DNA-labeled microtubules.

[Non-Patent Document 1]
H. Hess, et al., "Light-controlled Molecular Shuttles Made from Motor Proteins Carrying Cargo on Engineered Surfaces" Nano Letters, vol. 1, no. 5, pp. 235-239, 2001.

[Patent Document 1]
Japanese Patent Application Publication No. 2006-204241.

[Patent Document 2]
Japanese Patent Application Publication No. 2006-271323.

### SUMMARY OF THE INVENTION

Accordingly, it is an object of the present invention to provide a method of synthesizing a microtubule labeled with nucleotides without using a generally utilized biotin-avidin binding and also to provide methods for preserving and resynthesizing the produced microtubule.

In the present invention, to achieve the above object, (a) a microtubule stabilized after the polymerization is reacted with (b) a chemical crosslinking agent having a succinimide and maleimide and (c) nucleotides having a thiolated 3' or 5' end to synthesize a microtubule-chemical crosslinking agent-nucleotide complex.

As an example of the reaction, first, an amino group of the microtubule is reacted with succinimide of said chemical crosslinking agent to synthesize the microtubule-chemical crosslinking agent complex. Then, maleimide of the complex is reacted with said nucleotides having thiolated end to synthesize the microtubule-chemical crosslinking agent-nucleotide complex (nucleotide-labeled microtubule).

For said chemical crosslinking agent, it is preferable to use one of MBS (m-Maleimidobenzoyl-N-hydroxysuccinimide ester), Sulfo-MBS, SMPB (Succinimidyl 4-[p-maleimidophenyl] butyrate), Sulfo-SMPB, GMBS (N-[y-maleimidobutyryloxy] succinimide ester), Sulfo-GMBS, EMCS (N-[ε-maleimidocaproyloxy] succinimide ester), and Sulfo-EMCS. Molecular weights of these chemical crosslinking agents are about 280~460 Da, two orders of magnitude smaller than the molecular weights of avidins and streptavidins so that a range can be extended in which a modification ratio (labeling stoichiometry) of the single stranded DNAs to the microtubules is set.

For said nucleotides, by way of example, deoxyribonucleic acid (DNA) or ribonucleic acid (RNA) is used, which have a single stranded portion therein.

For preservation of the nucleotide-labeled microtubule thus synthesized, nucleotide-labeled tubulin is produced by cooling a solution of the nucleotide-labeled microtubule or adding a depolymerizing agent to the solution, and the solution of the tubulin is kept frozen after rapid freezing.

The preserved nucleotide-labeled microtubule can be resynthesized by mixing guanosine triphosphate (GTP) and a magnesium ion into the solution of the nucleotide-labeled tubulin and warming this mixture, which again causes a polymerization of the nucleotide-labeled tubulin and synthesis of the nucleotide-labeled microtubule.

As a preferable example, the nucleotide-labeled tubulin can be polymerized again by adding wild-type tubulin which is not labeled with nucleotides or tubulin which is labeled with nucleotides having different base sequences than said nucleotide-labeled tubulin into said mixture.

Furthermore, polymerization of the nucleotide-labeled tubulin and depolymerization of the nucleotide-labeled microtubule can be repeated several times before the rapid freezing of the nucleotide-labeled tubulin.

According to the invention, microtubule labeled with nucleotides can be synthesized without using a generally utilized biotin-avidin binding, thereby being able to set a labeling stoichiometry of the nucleotides to the microtubules arbitrarily.

Moreover, since it is possible to preserve and resynthesize the produced nucleotide-labeled microtubule, microtubules labeled with different kinds of single stranded nucleotides in a high density can be resynthesized, thereby increasing an orientation of the microtubules in patterning.

Such nucleotide-labeled microtubules increase efficiency and functionality in molecular transfer/molecular transport. In addition, these microtubules enable genetic diagnosis with high efficiency in sensing a specific DNA or RNA.

### BRIEF DESCRIPTION OF THE DRAWINGS

FIG.1 is a drawing showing a process of synthesizing a microtubule-chemical crosslinking agent complex;
FIG.2 is a drawing showing a process of synthesizing a microtubule-chemical crosslinking agent-nucleotide complex;
FIG.3A is a photograph of a fluorescence microscopy using a filter which passes fluorescence of TAMRA through;
FIG.3B is a photograph of the fluorescence microscopy using a filter which does not pass the fluorescence of TAMRA through; and
FIG.4 is a graph showing the results of gliding assays of the DNA-labeled microtubule in accordance with the examples of the invention and the wild-type microtubule.

### DETAILED DESCRIPTION OF THE PREFERRED EMBODIMENT

In the following, preferred embodiments of the present invention will be described with reference to the accompanying drawings. For examples below, Sulfo-GMBS was used as a chemical crosslinking agent and a single stranded deoxyribonucleic acid (ssDNA) with a thiolated 5' end as nucleotides.

### Example 1

A method of synthesizing a nucleotide-labeled microtubule as an embodiment of the present invention is following.

### (Preparation of microtubules)

In a solution of α, β-tubulins which were extracted and purified from porcine brains (40-60 µM), 1 mM GTP and 1 mM MgSO₄ were mixed. After the mixed solution was incubated at 37°C for 30 minutes, 80 µM Taxol was added to stabilize the polymerized microtubules and the resulting solution was let stand at room temperature.

### (Synthesis of microtubule-chemical crosslinking agent complexes)

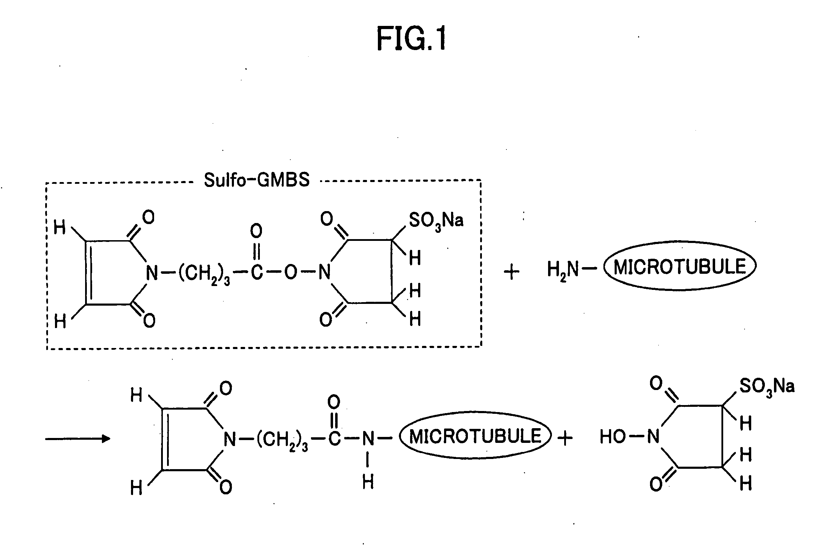

In the solution of microtubules prepared by the above procedures, 2.5 mM Sulfo-GMBS (22324, produced by PIERCE) was mixed, of which concentration was adjusted by, for example, anhydrous DMSO (dimethyl sulphoxide), and the mixed solution was further incubated under a shaded condition at room temperature for 30 minutes. During this procedure, as shown in FIG.1, an amino group of the microtubule was reacted with succinimide of the Sulfo-GMBS.

Then, after the incubated solution was centrifuged (at 30 krpm at 25 °C for 10 minutes) by an ultracentrifuge, the pellet recovered after centrifugation was rinsed by a buffer A (50 mM PIPES-KOH pH 7.0, 4 mM MgCl₂, and 20 µM Taxol) and suspended in the buffer A, thereby resulting in a solution of the microtubule-chemical crosslinking agent complex.

### (Synthesis of microtubule-chemical crosslinking agent-nucleotide complexes)

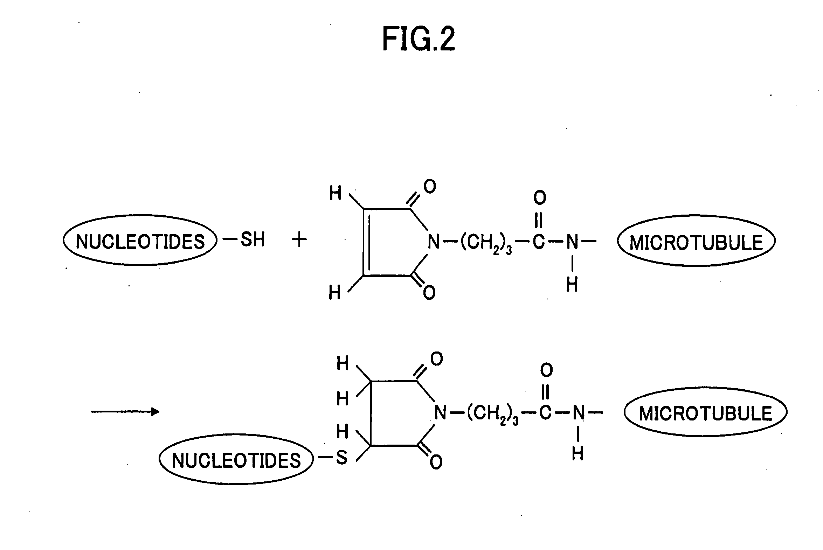

The solution of the microtubule-chemical crosslinking agent complexes prepared by the above procedures was mixed with the same concentration of ssDNAs having a thiolated 5' end, and the mixture was incubated under a shaded condition at room temperature for 90 minutes. During this incubation, as shown in FIG.2, maleimide of the Sulfo-GMBS labeled to the microtubule was reacted with thiol of ssDNA. Any strand length and base sequence of ssDNA can be used, and also ssDNA with a thiolated 3' end can be used. Furthermore, it is useful to label another end which is not thiolated with fluorescent dye such as TAMRA or FITC since the result of labeling the nucleotides to the microtubules can be checked visually with a fluorescent microscope. The ssDNA whose end is modified with thiol or fluorochrome can be obtained easily from entrusted companies such as SIGMA or OPERON. However, the ssDNA is mostly supplied with its ends bound to a protecting group in order to prevent a disulfide bond, so it is necessary to remove the protecting group by procedures which the company recommends prior to following the procedures of the invention. Moreover, when a concentration of the supplied ssDNAs is low, it is necessary to concentrate the solution by freeze-drying.

Then, 50 mM Tris-HCl pH 7.0 and 10 mM β mercaptoethanol of which concentrations were each adjusted by the buffer A were added to the incubated solution containing the complex, and the resulting solution was further incubated under a shaded condition at room temperature for 20 minutes.

After the incubated solution of the complexes was centrifuged (at 30 krpm at 25 °C for 10 minutes) by an ultracentrifuge, the pellet recovered after centrifugation was rinsed by and suspended in the buffer A, thereby resulting in a solution of the microtubule-chemical crosslinking agent-nucleotide complexes.

### (Evaluation of the synthesized nucleotide-labeled microtubules)

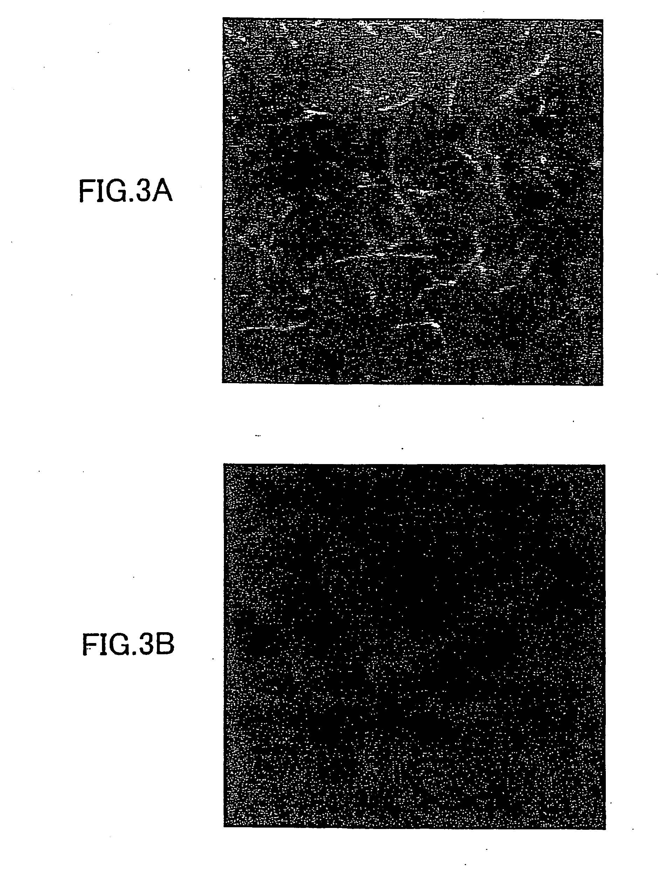

If either end of the nucleotide is labeled with the fluorochrome, it is possible to check visually the result of labeling the nucleotides to the microtubules by observing through a fluorescent microscope. For example, the fibrous microtubules can be identified, as shown in FIG.3A, when the result of labeling the ssDNAs of 10 bases, having a thiolated 5' end and a labeled 3' end with the fluorochrome, TARMA, to the microtubules is observed with a fluorescent microscope through a filter (WIG) which passes the fluorescence of TAMRA through. On the other hand, nothing appears, as shown in FIG.3B, when the result is observed with the fluorescent microscope through a filter (NIBA) which does not pass the fluorescence of TARMA through. Therefore, it can be visually determined whether the microtubules are labeled with the nucleotides.

From an experimental result from the measurements of absorbance or concentration of the microtubules, nucleotides, and chemical cross-linking agents, it is also possible to calculate a concentration ratio of the microtubules and nucleotides and quantify the labeling stoichiometry of the nucleotides to the microtubules. In the above example, the labeling stoichiometry was 0.61 meaning that at least one nucleotide chain was attached to one of the two molecules of tubulin heterodimer and the nucleotides were labeled to the microtubules in a quite high density. The labeling stoichiometry can be set arbitrarily by adjusting a concentration of the Sulfo-GMBS or nucleotides to be reacted with the microtubules which are already polymerized and stabilized.

A gliding speed of the nucleotide-labeled microtubules can be evaluated by a gliding assay, where the microtubules labeled with the nucleotides are caused to glide on a slide glass which motor proteins such as kinesin or dynein are adsorbed to.

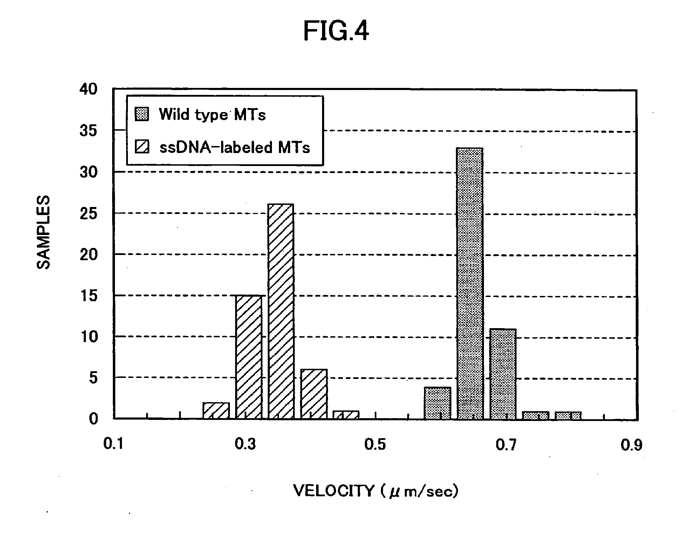

FIG.4 is a graph showing the result of the gliding assay of the wild-type microtubules (Wild type MTs) which were not labeled with the nucleotides and the microtubules (ssDNA-labeled MTs) which were labeled with the nucleotides at high density as described above. In this measurement, recombinant kinesin rk430 was used as motor protein, which was purified from E. coli, where kinesin expression plasmid derived from rats was expressed. Although the gliding speed of the microtubules labeled with the nucleotides at high density (ssDNA-labeled MTs) was reduced almost by half compared to the wild-type microtubules not labeled with nucleotides (wild type MTs), the microtubules labeled with the nucleotides at high density were capable of gliding smoothly. As the labeling stoichiometry of the nucleotides decreases, the difference of gliding speeds between the labeled and the wild-type microtubules becomes narrower.

It is also possible for the nucleotide-labeled microtubules to glide even if the motor proteins are exchanged for dyneins (e.g. HFB380) which can cause the nucleotide-labeled microtubules to glide with a speed several times faster than that of kinesins. In this case, the gliding speed easily exceeds 1.0 µm/s even if the microtubules are labeled with the nucleotides at high density.

### Example 2

Now, methods of preserving and resynthesizing the synthesized nucleotide-labeled microtubule will be described.

### (Depolymerization and preservation of the nucleotide-labeled microtubules)

The solution of the microtubule-chemical crosslinking agent-nucleotide complexes prepared by the above procedures was centrifuged (at 30 krpm at 25 °C for 10 minutes) by an ultracentrifuge. The pellet recovered after centrifugation was suspended in the ice-cold buffer B (80 mM PIPES-KOH pH 6.8, 1 mM MgCl₂, and 1 mM EGTA), and the suspension was incubated at 4 °C for 30 minutes. The nucleotide-labeled microtubules may also be depolymerized by adding a depolymerizing agent such as colcemids.

Then, the solution of the depolymerized microtubules was centrifuged (at 80 krpm at 2 °C for 10 minutes) by an ultracentrifuge, and the supernatant fluid was recovered. The solution to be centrifuged may be dispensed, if necessary. The recovered solution (nucleotide-labeled tubulins) was frozen by rapid freezing with liquid nitrogen and then kept in storage of liquid nitrogen. Also, before the rapid freezing of the nucleotide-labeled tubulins for preservation, the purity of the active nucleotide-labeled tubulins may be increased. This increase is accomplished by repeating several times a process of polymerizing the nucleotide-labeled tubulins by the following procedures and depolymerizing the polymerized nucleotide-labeled microtubules.

### (Resynthesis of the nucleotide-labeled microtubules)

The solution of the nucleotide-labeled tubulins preserved by the above procedures was thawed, in which the buffer B, 1 mM GTP, and 1 mM MgSO₄ were further added. After the resultant solution was mixed, it was incubated at 37 °C for 30 minutes. Glycerol may be added to the mixed solution to increase the efficiency of polymerization, if necessary. Moreover, the labeling ratio of the nucleotides to the microtubules can be set arbitrarily by mixing a moderate amount of wild-type tubulins which are not labeled with the nucleotides. Microtubules which are labeled with nucleotides having one of several kinds of base sequences can be synthesized by mixing a moderate amount of tubulins which are labeled with nucleotides having different base sequences than the above nucleotide-labeled tubulins.

Then, 80 µM taxol was added to the incubated solution to stabilize the polymerized microtubules. After the solution of the stabilized microtubules was centrifuged (at 30 krpm at 25 °C for 10 minutes) by an ultracentrifuge, the pellet recovered after centrifugation was suspended in the buffer A, thereby resulting in a solution of the resynthesized nucleotide-labeled microtubules.

Even though the invention has been described based on specific examples thereof, the invention is not limited to these examples. By way of example, in the above examples, Sulfo-GMBS was used as the chemical crosslinking agent, but the chemical crosslinking agent may comprise MBS (22311, produced by PIERCE), Sulfo-MBS (22312, produced by PIERCE), SMPB (22416, produced by PIERCE), Sulfo-SMPB (22317, produced by PIERCE), GMBS (22309, produced by PIERCE), EMCS (22308, produced by PIERCE), Sulfo-EMCS (22307, produced by PIERCE), and the like.

In addition, deoxyribonucleic acid (DNA) was used as the nucleotides in the above examples, but also ribonucleic acid (RNA) may be used.

## Claims

1. A method of synthesizing a nucleotide-labeled microtubule, **characterized by** comprising the step of:
causing a microtubule which is stabilized after polymerization to react with a chemical crosslinking agent which has succinimide and maleimide and nucleotides which have a thiolated 3' end or 5' end to synthesize a microtubule-chemical crosslinking agent-nucleotide complex.

2. The method of synthesizing a nucleotide-labeled microtubule as claimed in claim 1, **characterized by** further comprising the steps of:
synthesizing the microtubule-chemical crosslinking agent complex by causing an amino group of said stabilized microtubule to react with the succinimide of said chemical crosslinking agent; and
synthesizing said microtubule-chemical crosslinking agent-nucleotide complex by causing the maleimide of said microtubule-chemical crosslinking agent complex to react with said nucleotides.

3. The method of synthesizing a nucleotide-labeled microtubule as claimed in claim 1, **characterized in that** said chemical crosslinking agent is selected from:
MBS (m-Maleimidobenzoyl-N-hydroxysuccinimide ester), Sulfo-MBS, SMPB (Succinimidyl 4-[p-maleimidophenyl] butyrate), Sulfo-SMPB, GMBS (N-[y-maleimidobutyryloxy] succinimide ester), Sulfo-GMBS, EMCS (N-[ε-maleimidocaproyloxy] succinimide ester), and Sulfo-EMCS.

4. The method of synthesizing a nucleotide-labeled microtubule as claimed in claim 1, **characterized in that** said nucleotides are deoxyribonucleic acid (DNA) or ribonucleic acid (RNA) containing a single stranded portion.

5. A method of preserving a nucleotide-labeled microtubule, **characterized by** comprising the steps of:
depolymerizing the nucleotide-labeled microtubule as claimed in claim 1 to produce a nucleotide-labeled tubulin by cooling the solution of the microtubule-chemical crosslinking agent-nucleotide complex or adding a depolymerizing agent to said solution with the complex; and
rapid-freezing said nucleotide-labeled tubulin.

6. The method of preserving a nucleotide-labeled microtubule as claimed in claim 5, **characterized by** further comprising the step of:
repeating the polymerization of said nucleotide-labeled tubulin and the depolymerization of said nucleotide-labeled microtubule several times before proceeding with said rapid-freezing.

7. A method of resynthesizing a nucleotide-labeled microtubule, **characterized by** comprising steps of:
mixing a guanosine triphosphate (GTP) and magnesium ion into the solution of the rapidly frozen nucleotide-labeled tubulin as claimed in claim 5 or.claim 6; and
heating said mixture to polymerize said nucleotide-labeled tubulin again, thereby resynthesizing said nucleotide-labeled microtubule.

8. The method of resynthesizing a nucleotide-labeled microtubule as claimed in claim 7, **characterized by** further comprising the step of:
adding wild-type tubulin which is not labeled with nucleotides into said mixture.

9. The method of resynthesizing a nucleotide-labeled microtubule as claimed in claim 7, **characterized by** further comprising the step of:
adding tubulin labeled with nucleotides, having a different base sequence than said nucleotide-labeled tubulin which is frozen by the rapid-freezing, into said mixture.

## Patentansprüche

1. Verfahren zum Synthetisieren eines nukleotidmarkierten Mikrotubulus, **gekennzeichnet durch** folgenden Schritt:
Veranlassen, dass ein Mikrotubulus, der nach Polymerisierung stabilisiert ist, mit einem chemischen Quervernetzungs-Wirkstoff reagiert, der Succinimid und Maleimid und Nukleotide hat, welche ein Thiolat-3'-Ende oder ein -5'-Ende haben, um einen mikrotubulus-chemischen Quervernetzungs-Wirkstoff Nukleotid-Komplex zu synthetisieren.

2. Verfahren zum Synthetisieren eines nukleotidmarkierten Mikrotubulus nach Anspruch 1, **dadurch gekennzeichnet, dass** es weiter die folgenden Schritte umfasst:
Synthetisieren des mikrotubulus-chemischen Quervemetzungs-Wirkstoff-Komplexes, indem veranlasst wird, dass eine Amino-Gruppe des stabilisierten Mikrotubulus mit dem Succinimid des chemischen Quervernetzungs-Wirkstoffs reagiert: und
Synthetisieren des mikrotubulus-chemischen Quervernetzungs-Wirlcstoff Nulkleotid-Komplexes, indem veranlasst wird, dass das Maleimid des mikrotubulus-chemischen Quervernetzungs-Wirkstoff Komplexes mit den Nukleotiden reagiert.

3. Verfahren zum Synthetisieren eines nukleotidmarkierten Mikrotubulus nach Anspruch 1, **dadurch gekennzeichnet, dass** der chemischen Quervemetzungs-Wirkstoff ausgewählt wird von:
MBS (m-Maleimidobenzoyl-N-Hydroxysuccinimid-Ester), Sulfo-MBS, SMPB (Succinimidyl-4-[p-Maleimidophenyl]-Butyrat), Sulfo-SMPB, GMBS (N-[γ-Maleimidobutyryloxy]-Succinimid-Ester), Sulfo-GMBS, EMCS (N-[ε-Maleimidocaproyloxy] - Succinimid-Ester), und Sulfo-EMCS.

4. Verfahren zum Synthetisieren eines nukleotidmarkierten Mikrotubulus nach Anspruch 1. **dadurch gekennzeichnet, dass** die Nukleotiden Deoxyribonukleid-Säure (DNA) oder Ribonukleid-Säure (RNA) sind, welche einen einfach verketteten Bereich enthalten.

5. Verfahren zum Bewahren eines nukleotidmarkierten Mikrotubulus, **dadurch gekennzeichnet, dass** dies folgende Schritte umfasst:
Depolymerisierung des nukleotidmarkierten Mikrotubulus nach Anspruch 1, um ein nukleotidmarkiertes Tubulin zu erzeugen, indem die Lösung des mikrotubulus-chemischen Quervemetzungs-Wirkstoff-Nukleotid-Komplexes abgekühlt wird oder ein Depolymerisierungs-Wirkstoff der Lösung mit dem Komplex hinzugefügt wird; und
schnelles Einfrieren des nukleotidmarkierten Tubulins.

6. Verfahren zum Bewahren eines nukleotidmarkierten Mikrotubulus nach Anspruch 5, **dadurch gekennzeichnet, dass** dies außerdem folgenden Schritt umfasst:
Wiederholen der Polymerisierung des nukleotidmarkierten Tubulins und der Depolymerisierung des nukleotidmarkierten Mikrotubulus mehrere Male vor dem Weitergehen mit dem Schnell-Einfrieren.

7. Verfahren zum Resynthetisieren eines nukleotidmarkierten Mikrotubulus, **dadurch gekennzeichnet, dass** dies folgende Schritte umfasst:
Mischen eines Guanosin-Triphosphats (GTP) und Magnesium-Ions in die Lösung des schnell-gefroren nukleotidmarkierten Tubulins nach Anspruch 5 oder 6; und
Erhitzen der Mischung, um das nukleotidmarkierte Tubulin wiederum zu polymerisieren, um **dadurch** den nukleotidmarkierten Mikrotubulus zu resynthetisieren.

8. Verfahren zum Resynthetisieren eines nukleotidmarkierten Mikrotubulus nach Anspruch 7, **dadurch gekennzeichnet, dass** dies außerdem folgenden Schritt umfasst:
Hinzufügen von wildem Tubulin, welches nicht mit Nukleotiden markiert ist, in die Mischung.

9. Verfahren zum Resynthetisieren eines nukleotidmarkierten Mikrotubulus nach Anspruch 7, **dadurch gekennzeichnet, dass** dies außerdem folgenden Schritt umfasst:
Hinzufügen von Tubulin, welches mit Nukleotiden markiert ist, welche eine andere Basissequenz als das nukleotidmarkierte Tubulin hat, welches durch Schnell-Frieren gefroren ist, in die Mischung.

## Revendications

1. Procédé pour la synthèse d'un microtubule à étiquette nucléotide, **caractérisé par** le fait de comprendre l'étape consistant:
à faire réagir un microtubule qui est stabilisé après une polymérisation avec un agent de réticulation chimique qui possède un succinimide et un maléimide et des nucléotides qui possèdent une extrémité 3' ou une extrémité 5' thiolée pour synthétiser un complexe de microtubule-agent de réticulation chimique-nucléotides.

2. Procédé pour la synthèse d'un microtubule à étiquette nucléotide selon la revendication 1, **caractérisé par** le fait de comprendre en outre les étapes:
de synthèse du complexe de microtubule-agent de réticulation chimique en faisant réagir un groupe amino dudit microtubule stabilisé avec le succinimide dudit agent de réticulation chimique; et
de synthèse dudit complexe de microtubule-agent de réticulation chimique-nucléotides en faisant réagir le maléimide dudit complexe de microtubule-agent de réticulation chimique avec lesdits nucléotides.

3. Procédé pour la synthèse d'un microtubule à étiquette nucléotide selon la revendication 1, **caractérisé en ce que** ledit agent de réticulation chimique est choisi parmi:
MBS (ester de m-maléimidobenzoyl-N-hydroxysuccinimide), sulfo-MBS, SMPB (4-[p-maléimidophényl]butyrate de succinimidyle), sulfo-SMPB, GMBS (ester de N-[γ-maléimidobutyryloxy]-succinimide), sulfo-GMBS, EMCS (ester de N-[ε-maléimidocaproyloxy]succinimide) et sulfo-EMCS.

4. Procédé pour la synthèse d'un microtubule à étiquette nucléotide selon la revendication 1, **caractérisé en ce que** lesdits nucléotides sont un acide désoxyribonucléique (ADN) ou un acide ribonucléique (ARN) contenant une portion à un seul brin.

5. Procédé pour la préservation d'un microtubule à étiquette nucléotide, **caractérisé par** le fait de comprendre les étapes:
de dépolymérisation du microtubule à étiquette nucléotide selon la revendication 1 pour produire une tubuline à étiquette nucléotide en refroidissant la solution du complexe de microtubule-agent de réticulation chimique-nucléotides ou en ajoutant un agent de dépolymérisation à ladite solution avec le complexe; et
de congélation rapide de ladite tubuline à étiquette nucléotide.

6. Procédé pour la préservation d'un microtubule à étiquette nucléotide selon la revendication 5, **caractérisé par** le fait de comprendre en outre l'étape:
de répétition de la polymérisation de ladite tubuline à étiquette nucléotide et de la dépolymérisation dudit microtubule à étiquette nucléotide plusieurs fois avant de poursuivre avec ladite congélation rapide.

7. Procédé pour la re-synthèse d'un microtubule à étiquette nucléotide, **caractérisé par** le fait de comprendre les étapes:
de mélange d'un guanosine triphosphate (GTP) et d'ion magnésium dans la solution de la tubuline à étiquette nucléotide congelée rapidement selon la revendication 5 ou la revendication 6; et
de chauffage dudit mélange pour polymériser à nouveau ladite tubuline à étiquette nucléotide, resynthétisant ainsi ledit microtubule à étiquette nucléotide.

8. Procédé pour la re-synthèse d'un microtubule à étiquette nucléotide selon la revendication 7, **caractérisé par** le fait de comprendre en outre l'étape:
d'ajout d'une tubuline de type sauvage qui n'est pas marquée avec des nucléotides dans ledit mélange.

9. Procédé pour la re-synthèse d'un microtubule à étiquette nucléotide selon la revendication 7, **caractérisé par** le fait de comprendre en outre l'étape:
d'ajout d'une tubuline marquée avec des nucléotides, présentant une séquence de bases différente de celle de ladite tubuline à étiquette nucléotide qui est congelée par la congélation rapide, dans ledit mélange.
